# EUROPEAN PATENT APPLICATION

(11) **EP 4 761 173 A1**
(43) Date of publication of application: **17.06.2026**
(21) Application number: 24218606.2
(22) Date of filing: 10.12.2024
(51) Int. Cl.: H04L 9/40, H04L 65/1108, H04L 67/025, H04L 67/12, G16H 40/40, G16H 40/67

(54) **ENABLING REMOTE SERVICEABILITY OVER THE INTERNET**

(71) Applicant: Koninklijke Philips N.V., 5656 AE Eindhoven (NL)
(72) Inventor: CHALUVANAHALLI RUDRAPPA, Deepak, Eindhoven (NL); RAMASAMY, Jaikumar, Eindhoven (NL); CHANDRASHEKARA, Manohar, 5656AG Eindhoven (NL); CHILLAPALLI, Jyothi, Eindhoven (NL)
(74) Representative: Philips Intellectual Property & Standards

(57) **Abstract**

There is provided a method for remote servicing and technical support of imaging equipment that involves obtaining information from a local node with at least one imaging system is disclosed. The method establishes a two-way secure communication channel between a local and a remote node operator, wherein identity confirmation of the remote operator occurs through a database, granting access to the local node's imaging system peripheral devices upon approval. The method includes generating media events transmitted to the remote node, transmitting information streams via a e.g. WebRTC channel, and allowing remote operators to access multiple local systems. The method also involves capturing local node screens with a virtual camera module and transmitting peripheral device inputs to the remote node. A receiver module on the remote node receives shared screens and emulated inputs, enabling remote change requests through keyboard and mouse events.

## Description

### FIELD OF THE INVENTION

The present document relates to healthcare technology, specifically to remote serviceability solutions for imaging systems using secure internet-based communication protocols.

### BACKGROUND OF THE INVENTION

In healthcare technology, imaging systems are frequently deployed at customer locations. Yet, the usage of these systems is quite intense, which might lead to unexpected problems and technical faults with the imaging machines, which disrupts the workflow and can lead to significant financial losses to hospitals. Furthermore, service engineers may not be available on-demand due to e.g., a trip to another town. Hence, timely resolvement of these issues is needed. Furthermore, these systems require maintenance to ensure functionality and efficiency.

Traditional methods for remote access include utilizing the customer's VPN or implementing third-party remote connection software. Both approaches present challenges, such as stringent security requirements for VPNs and security concerns with third-party software, especially in a healthcare setting.

Existing solutions often fail to provide a secure and efficient method for maintenance without compromising the customer's network security. The need for a streamlined solution that allows secure remote access without relying on VPNs or third-party software remains unmet. This gap highlights a new approach to enable maintenance over the internet with enhanced security and authorization protocols.

### SUMMARY OF THE INVENTION

A disadvantage of the known methods is that they are not applicable in medical imaging practice due to e.g., strict regulatory concerns and privacy concerns. Existing approaches fail to establish a secure communication that allows to make the changes on the local system in an efficient and secure manner to maintain the operation of the machine. Furthermore, existing systems fail to establish an on-demand connection in clinical practice in case there is such a need.

It is an objective of the invention to provide a method, system, non-transitory omputer readable medium, computer program that allows to solve the above-mentioned challenges in clinical practice and provide an improved apparatus for remote technical support and remote servicing in clinical practice.

According to a first aspect of the invention, there is provided a method for remote servicing and technical support of imaging systems, the method comprising: obtaining operational information of a local node, the local node comprising at least one imaging system and at least one control interface; establishing a two-way secure communication channel between an operator of a remote node and an operator of the local node, wherein an identity confirmation request of the operator of the remote node is performed; confirming the identity of the operator of the remote node through the identity confirmation request, transmitting the operational information of the local node to the remote node and granting remote service access to the local node, the remote service access to the local node comprises granting access to at the at least one control interface allowing to execute remote servicing to the at least one imaging system, wherein an approval request of such remote servicing execution is displayed to the local operator. In some embodiments, the approval request is optional.

In a second aspect of the invention, there is provided a non-transient/non-transitory computer readable medium containing program instructions for causing a computer to perform the method of: obtaining operational information of a local node, the local node comprising at least one imaging system and at least one control interface; establishing a two-way secure communication channel between an operator of a remote node and an operator of the local node, wherein an identity confirmation request of the operator of the remote node is performed; confirming the identity of the operator of the remote node through the identity confirmation request, transmitting the operational information of the local node to the remote node and granting remote service access to the local node, the remote service access to the local node comprises granting access to at the at least one control interface allowing to execute remote servicing to the at least one imaging system, wherein an approval request of such remote servicing execution is displayed to the local operator.

In a third aspect of the invention, there is provided a system, the system configured for obtaining operational information of a local node, the local node comprising at least one imaging system and at least one control interface; establishing a two-way secure communication channel between an operator of a remote node and an operator of the local node, wherein an identity confirmation request of the operator of the remote node is performed; confirming the identity of the operator of the remote node through the identity confirmation request, transmitting the operational information of the local node to the remote node and granting remote service access to the local node, the remote service access to the local node comprises granting access to at the at least one control interface allowing to execute remote servicing to the at least one imaging system, wherein an approval request of such remote servicing execution is displayed to the local operator.

In a fourth aspect of the invention, there is provided a computer program (product) comprising instructions which, when executed by a processing system, cause the processing system to carry out the method as described according to any of the embodiments or any combination of the embodiments according to the present invention.

These and other aspects of the invention will be apparent from and elucidated with reference to the embodiment(s) described hereinafter.

### BRIEF DESCRIPTION OF THE DRAWINGS

In order to describe the manner in which the advantages and features of the disclosure can be obtained, a more particular description of the principles briefly described above will be rendered by reference to specific embodiments thereof which are illustrated in the appended drawings. Understanding that these drawings depict only exemplary embodiments of the disclosure, and are not therefore to be considered to be limiting of its scope, the principles herein are described and explained with additional specificity and detail through the use of the accompanying drawings in which:
Fig. 1 depicts a first embodiment according to the invention;
Fig. 2 depicts a second embodiment according to the invention;
Fig. 3 depicts a third embodiment according to the invention.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The present application describes various embodiments of a system, method, computer program and non-transitory computer readable medium that is used for establishing a remote servicing connections and/or allows for remote servicing of e.g., an imaging device of a local node with the support of a secured connection using various tools for establishing such a remote servicing device.

A Virtual Private Network (VPN) is a protocol that creates a secure and encrypted connection over a less secure network, such as the internet. It allows users to send and receive data as if their devices were directly connected to a private network, ensuring privacy and security. VPNs are commonly used to protect sensitive data, maintain privacy, and access restricted resources by masking the user's IP address and encrypting data transmissions. VPN requires separate installations and security measures, which in healthcare setting may prove cumbersome.

While VPNs offer several benefits, they also come with some disadvantages, that are difficult to overcome, especially in a healthcare setting: 1. Reduced Speed: VPNs can slow down internet connection due to the encryption process and the distance to the VPN server. 2. Cost: Many reliable VPN services require a subscription, which can be a financial consideration for users. 3. Compatibility Issues:
Some healthcare sites and applications that are used for communication and servicing may not function properly when accessed through a VPN, leading to potential accessibility problems. 4. Data Logging: Not all VPN providers are trustworthy; some may log user data or have inadequate privacy policies, potentially compromising anonymity, which is a big concern in a healthcare setting. 5. Complexity:
Setting up and configuring a VPN can be complicated for some users, especially those who are not tech-savvy, which is especially the case with at least some healthcare workers. 6. Legal and Policy Risks: In some countries, using a VPN may violate local laws, and users could face penalties for accessing restricted content, which is especially applicable due to concerns on patient data and patient privacy. 7. Trust Issues: Users must trust their VPN provider with their data; if the provider is compromised or has poor security practices, this can pose risks.

The present application is targeted to solving the challenges that are posed by VPN use, especially in a healthcare setting. On the other hand, maintaining e.g. imaging equipment is crucial for ensuring accurate results and prolonging the lifespan of the devices and not to have emergency situations, which will affect patient health in clinical settings. Maintenance of imaging equipment is essential for several reasons. One of the reasons is Accuracy and Reliability: Regular maintenance ensures that imaging equipment operates correctly, providing accurate and reliable results. This is critical in fields like healthcare, where precise imaging is vital for diagnosis and treatment. Another reason is that maintenance of equipment prolongs equipment lifespan: Routine care helps extend the lifespan of the equipment by preventing wear and tear and addressing minor issues before they become major problems. Another reason is cost-effectiveness: Preventive maintenance can reduce the likelihood of costly repairs or replacements. It helps identify issues early, minimizing downtime and unexpected expenses. Yet another reason is quality of results: Regular calibration and cleaning maintain the quality of the images produced, which is crucial for applications like medical imaging, research, and industrial inspections. Also, patient safety is a big factor: Regular maintenance of equipment allows to avoid problems with equipment in clinical settings.

Maintenance of imaging equipment is usually done once a quarter or on a bi-annual basis. However, due to intensive use of imaging equipment, more frequent maintenance might be needed. This presents challenges due to insufficient number of maintenance engineers, strict schedules and high costs associated with such requests. On the other hand, each problem with imaging equipment, even if very minor one, might influence the whole workflow of the department due to the need for re-assigning patients to other places. Hence, proactive and timely maintenance is key. Some of the problems can be solved by local staff under the guidance of the service engineer. However, to do so, there are many challenges unique to hospital setting thta need to be solved. This application provides for methods, systems, computer programs, non-volatile mediums for solving the needs of remote servicing in clinical settings, especially in radiology settings.

In healthcare solutions, hardware systems are frequently deployed at customer locations to interact with existing systems. These systems require seamless integration with the customer's network, often necessitating remote servicing for maintenance and support. Traditional methods for remote servicing include utilizing the customer's VPN or implementing third-party remote software. Each approach presents distinct challenges that complicate effective remote servicing.

Utilizing the customer's VPN for remote access demands stringent security measures, including comprehensive monitoring services. This method is suitable when multiple systems are deployed within the customer network, and the customer consents to establish a VPN connection with the vendor. However, many customers are reluctant to share their VPN due to security concerns. Alternatively, third-party remoting software, such as Team Viewer or VNC, offers another option. Yet, customers often hesitate to deploy these software components, fearing potential security vulnerabilities.

The described method addresses these challenges by providing a secure remote connection solution over the internet, eliminating the need for VPN access. This can be particularly useful in a healthcare setting, such as a hospital. This method at least in some embodiments employs a communication protocol, such as WebRTC, to establish a two-way secure communication channel, facilitating the sharing of screens, keyboard, and mouse inputs. The system may include a virtual camera module that captures the local system's screen, transmitts the local system's screen to a transmitter module, the transmitter module converts the input screens into a media stream, which is published over the communication channel. Authorized remote users join the channel using a receiver module, receiving and rendering the shared screen media events on their web application layout. This configuration at least in some embodiments may enable remote screen sharing and control, combining communication with screen capture, keyboard and mouse emulation, media construction, and authentication and authorization techniques.

In a first aspect of the invention, a method, system, computer program, non-transitory computer-readable medium is described configured for remote servicing and technical support of imaging equipment. As an illustrative example, a method is further described. In a first aspect of the invention, the method comprises obtaining operational information of a local node, the local node comprising at least one imaging system and at least one control interface; establishing a two-way secure communication channel between an operator of a remote node and an operator of the local node, wherein the operator of the local node establishes the requirements for such a session and requests an identity confirmation of the operator of the remote node; and confirming the identity of the operator of the remote node and upon a matching identity check, operational information of the local node is transmitted to the remote node and remote service access to the local node is granted, the remote service access to the local node includes granting access to at least one local nodes' imaging system peripheral device, allowing to execute the changes to the local nodes' imaging system. In some embodiments, such changes need to be approved by the operator of the local node. However, in other embodiments, there is no need for approval of such changes. In yet other embodiments, such changes are approved by a hospital administrator and/or by another remote operator, such as a qualified service engineer.

It is to be understood that operational information refers to data that is directly related to the operation of the machine. This data can include a wide range of information, such as: 1. system configuration and settings: Information about the imaging system's setup, including software configurations, hardware specifications, resolution settings, and other customizable parameters (e.g., exposure time, contrast, scanning speed). 2. system usage data: Details on how the imaging system is being used, including the number of scans or captures performed, operator logs, imaging modes or protocols in use, and any adjustments made during operation. 3. performance metrics: data that tracks the performance of the imaging system, such as image quality (resolution, clarity, noise levels), scan times, throughput (how many images or scans per unit time), and error rates. 4. maintenance and calibration information: details regarding regular system maintenance schedules, calibration procedures, and any adjustments or repairs that need to be made to ensure the imaging system is operating at peak performance. 5. error logs and troubleshooting data: logs that record any errors, faults, or malfunctions in the imaging system, including system alerts, diagnostic codes, and steps taken to resolve issues. 6. resource availability and workflow information: information about the availability of imaging consumables (e.g., film, contrast agents), hardware components (e.g., detectors, lenses), current workflow, and software updates or patches. 6. user access and permissions: data on who has access to the system, including operator roles, user credentials, and permissions to configure, operate, or modify system settings. 7. compliance and regulatory data: Information related to adherence to regulatory standards, such as safety protocols, image storage policies (e.g., HIPAA in healthcare), and maintenance logs for compliance audits.

It is further understood that the local node may refer to e.g., a local sight that may comprise at least one (e.g., one, two, three, etc.) imaging systems with appropriate peripheral devices, such as control peripherals (workstation having a keyboard and a mouse), peripheral equipment (e.g, bolus devices) and other equipment that may influence the operation of the machine. At least in some embodiments, establishing a two-way communication includes establishing a secure connection through e.g., VPN. The operator of the local node may establish requirements of the secure connection and secure session, including but not limited to: the level of access that the remote operator has, service level, security level, duration of the session, electing identities of specific remote operators, etc. The operator then confirms the identity upon a matching identity check by e.g., confirming the identity visually, through an encrypted cryptographic key, through ID scan and by other means suitable in the field. In some embodiments, the operator of the local node may request a specific operator of the remote node to join the session. In some embodiments, identity check is being performed, the identity check may exemplary comprise taking a selfie by the operator of the and sending it to a local operator for a confirmation of the identity. Upon a matching identity check, operational information of the local node is transmitted to the remote node and remote service access to the local node is granted. In this way, a secure connection is established ensuring that only qualified personnel has access to the system and none of the private data (e.g., patient data or scan parameters) are being shared with unauthorized personnel. The remote service access to the local node includes granting access to at least one local nodes' imaging system peripheral device, allowing to execute the changes to the local nodes' imaging system, which may need to be approved by the operator of the local node. Exemplary, the operator of the remote node is sending the operational commands to control interface of the local node, wherein the control interface may comprise any perfipheral devices, such as keyboards, wherein upon sending the control commands, the control interface executes the change to the local device through, e.g., the peripheral device by emulating the received commands. It is to be understood that the local node has a broad definition, and may include imaging device (-s), control interface (-s) and peripheral devices (such as PACS database connected to the imaging system, arhiver, mouse, etc).

In some embodiments, local servicing may refer to making changes on the system via an engineering level access that allows to make diagnostic of the medical imaging system (s) of the local node and make necessary changes to the remote medical imaging system. The advantage of this embodiment is that the remote operator may have immediate service access to the local node, whereas the changes are being done under the supervision of the local node operator. This way, the e.g., service engineer does not need to travel to the e.g., hospital to make the necessary changes, but can make the necessary changes remotely, while a communication channel may be established to communicate and coordinate the servicing locally so that the local operator can intervene if there is such a need. This also can serve as an educational endeavor for the local operator.

In some embodiments, the remote servicing access may refer to establishing only remote access of the local node that has the control interface to an imaging system from a remote node. In this embodiment, the remote operator has access to the local node through e.g., sharing the screen of the local nodes' imaging device and/or control interface for checking the changes using existing user access level (or upgrading to service engineer access level), so that the remote operator can read the screen of the local device and operate the local nodes' imaging device through the screen. In some embodiments, the operation is done through emulating of the commands.

In some embodiments, the identity confirmation request comprises a comparison with a database of operators, and wherein any one of or combination of the following devices are used for execution of the identity confirmation request of the operator of the remote node: camera, fingerprint sensor, ID scan device, RFID scan device, database verification unit, knowledge-based authentication system. Exemplary, each hospital may have a database of "trusted experts", which may have different titles: such as a power-user, service engineer, senior service engineer, manager of service department and others, and by using a specific database for e.g., a specific hospital, only qualified people for that hospital may have access to the imaging systems of the local node. As another example, the database may have only qualified engineers of a specific vendor, and by performing a comparison, restriction to only desired personnel is performed. Exemplary, the trusted expert may insert his ID in the ID scan device in addition to entering his private password in order to perform the identification.

In some embodiments, establishing the two-way secure communication channel comprises confirmation by an administrator and/or the local operator of a remote service access request, and wherein each confirmed and unconfirmed remote service access request is stored in a service database as a log for further review by the administrator and/or the local operator. In this way, exemplary, an efficient workflow can be established that allows to utilize the resources that are available at the moment (e.g., the operator of the remote node is a super-user, but is not a service engineer), wherein the operator of the remote node is allowed to perform service changes, to a limited or essentially unlimited extent, but then the service engineer, upon a routine check of the system, can detect what changes have been made, and revert changes if needed.

In some embodiments, at least one media event is generated and transmitted to the remote node, the media event comprising at least one of: capturing of a screen of the local nodes' imaging device by a virtual camera module, capturing of the screen of the local nodes' imaging device by a camera module, screen scraping of the local screen of the local nodes' imaging device, capturing control interface inputs and emulatuing such interface inputs to the remote node via an emulation module. Exemplary, the virtual camera module captures the screen (e.g., desktop of a monitor) of the imaging system, imaging device or any peripheral device, such as a PACS system connected to the imaging system, of the local node. Then upon capturing of the screen, the information on the screen is being transmitted to the remote node and emulated on the remote nodes' screen (e.g., desktop of a monitor). In this way, the operator of the remote node, upon such a need, may see what is happening on the local screen of an imaging device. This can be performed in real-time or not in real-time (e.g., screen information is transmitted and stored on the remote node for further review at some point in time). It is to be understood that an emulation device is a program, system or device that enables a computer system to behave like another device, e.g., imaging system of the local node is being emulated on a remote node. An emulation device allows one computer system (aka "the host") to imitate the functions of another (aka "the guest"). In the present examples, the remote node is the host, while the at least one imaging system of the local node is the guest.

In some embodiments, the operational information of the local node is transmitted to the operator of the remote node using a WebRTC channel, wherein a near real-time data exchange between the operators of the local and remote nodes is established using the WebRTC channel. It is to be understood that WebRTC is the preferred embodiment due to possibilities of integrating the video of the local system to the workflow, which allows for the operator of the remote node and the operator of the local node to communicate with each other. WebRTC open source enables real-time audio, video, and data sharing directly within web browsers or within a certain application. Hence, it allows for a robust communication without needing additional plugins or installations, and can be hosted both on-prem and in the cloud. As a result, using WebRTC has certain advantages in the remote on-the-fly servicing of the imaging systems. However, other protocols, in addition or as an alternative, to the WebRTC protocol can be used. A non-limiting list includes such protocols, software development kits (SDK) and communication protocols as: VideoSDK, Twilio, MirrorFly, Agora, Jitsi, Vonage, AWS Chime SDK, Enablex, Whereby, SignalWire. As an example, use of Twilio in addition to the WebRTC protocol may allow to manual configure additional codebase that would allow for utilizing multiple audio and video inputs, from multiple local and/or remote nodes, such as multiple local nodes with one remote node, multiple remote nodes with one local node, or multiple local nodes with multiple remote nodes. Furthermore, Twilio's call insights feature allows error tracking and analysis, which would have additional advantages in remote servicing context, such as tracking for additional errors that are produced due to e.g., bandwith or camera problems, which would allow to track peripheral device faults, such as camera faults, which don't directly influence the operation of the imaging system, but which may give such an impression to the operator of the remote node.

In some embodiments, the method further includes tracking screen status of the local operator, the screen status shared with the operator of the remote node, and wherein the service access to at least one imaging system is executed via least one control interface by the operator of the remote node.

In some embodiments, The method of any one of the previous claims, wherein the remote service access to the operator of the remote node includes granting remote service access to multiple imaging systems of the local node or multiple affiliated local nodes, wherein each of the multiple imaging systems comprises at least one control interface. Exemplary, the remote node user that obtained access to the local node has access to multiple imaging systems of the local node, such as multiple imaging systems that are installed in a specific hospital and connected by a hospital network. Hence, in this embodiment, the operator of the remote node can proactively analyze any issues that may arise with respect to any imaging device of a local node. Affiliated nodes may comprise local nodes of one hospital, local nodes of multiple hospitals affiliated with each other (such as a University Hospital having multiple sites within the same city or within multiple cities), or a hospital chain (such as multiple hospitals spread across the country or different countries), or other examples. It is to be understood that hospitals is merely exemplary, and this can refer to imaging centers, local sites, airport security services, and others.

In some embodiments, a serviceability unit may be used. A serviceability unit may refer to a processor that allows to change the operational settings of the device, a specific system that allows to change the operational settings of the device, such as a dedicated service computer with appropriate hardware, and any other systems that may influence the operation of the imaging device and may or may not have service-level access. Exemplary, the serviceability unit is initialized, the serviceability unit configured to change the operational settings of the at least one imaging device through the two-way secure communication channel. Exemplary, changing operational settings may refer to changing scan protocol (e.g., substituting one protocol with another from a database of protocols or a customary protocol), changing scan parameters (e.g., for CT imaging device: kV and mAs and slices and reconstruction algorirhms, for MRI machine: T1/T2, for digital x-ray device and angiography devices: mAs and kV). It is to be understood that it is an exemplary explanation, and other examples are envisioned, such as changing the reconstruction protocols, de-activating certain components within the imaging device or activating certain components within the imaging device.

In some embodiments, the operational settings change needs to be approved by operators other than the local operator and the remote operator. For instance, if is a critical change, the remote node operator may include a remote node operator with a higher access level. For example, in case of an imaging device super-user (advanced level clinical technician), the technician may make changes to the locel imaging node, but these changes would require an approval from the service engineer, which may be done in real-time or not. Essentially, this provides a fail-safe mechanism, which allows to make necessary changes to continue hospital workflow uninterrupted, but may also prevent inadvertent changes in case the remote node operator lacks experience with a specific issue. For instance, this could be an exampel of an imaging device super-user requiring approval from a service engineer, a service engineer requiring approval from another service engineer, a service engineer requiring approval from a hospital admininstrator, an imaging device super-user requiring approval from the hospital administrator, etc.

In some embodiments, the operational information of the local node comprises any one of: service logs, scan information, patient images , service images, reconstruction algorithm information, operator access logs, wherein optionally an analytics module is used for analysing the operational information for potential technical faults of the at least one imaging system. It is to be understood that these are merely examples, and other information can be envisioned within the current application, which may help in identifying problems with the imaging device. For instance, transmitting patient images may allow to spot artifacts, which the local operator may not be able to spot, and by this deduce the technical fault associated with the equipment (such as: detector degradation). In some embodiments, the patient data (and other data) is anonymized or pseudo-anonymized. This way the potential technical faults may be determined.

In some embodiments, the potential technical faults are visualized or otherwise communicated to at least one of: local operator, remote operator and/or administrator, wherein an instruction for correction of such potential technical faults is provided, wherein upon confirmation of such correction, the system analyses if the potential technical fault is still present, and in the affirmative event, proposes additional corrective actions. Otherwise communicated may refer to the way the potential technical faults are being presented. For ease of understanding, the potential technical faults may be visualized, such as visualized on a 3D model of the imaging device and indicating where the technical faults probably occurs. However, other approaches of conveying the information may be used, such as in textual, tabular, audial, audio-visual and other forms of communication. It is to be understood that in this embodiment, the system may suggest certain corrective actions through e.g., an instruction taken from a service manual, wherein the user is referred directly to relevant pages and/or the pages are being showed to e.g., the local/remote operator. Then the operator makes the proposed corrective action of a possible imaging device problem (correction of potential technical faults), and confirms that the corrective action has taken place. Then, upon such a confirmation, the system makes an additional check in order to confirm that the possible imaging device problem has been solved. If yes, then the system provides such a notification to the operators. If not, then the system provides other corrective actions that may be applicable in the example in an iterative manner until the problem is solved, or until it is determined that e.g., a service check is required.

In some embodiments, the operational information is being continuously monitored, and wherein the remote and/or local operator receive an alert of any possible technical faults that may affect the operation of the at least one imaging system of the local node. Exemplary, an analysis unit, such as an analysis unit comprising multiple dedicated processors with embedded code, is deployed in the local node (be it in the medical imaging device or the control interface or both), and analyze any possible technical faults with the medical imaging device. In some embodiments, this could be a digital twin system that makes a digital representation for further comparison and analysis. In some embodiments, the multiple processors are scattered around critical components (like gantry in the CT machine) and constantly analyze the technical faults associated with a specific component. Then the gathered information is either analyzed on-premises in order to identify possible faults, and upon such a determination, transmitting the information related to the faults to a remote system, and/or the information is directly transmitted to the remote node and/or 3^{rd} party cloud for further analysis and determination of possible technical faults. In some embodiments, a feed mechanism is deployed for transmitting the data from the analysis unit to the remote node.

In some embodiments, if a critical technical fault/critical technical faults are detected, a critical alert is generated to the operator of the remote node, the operator of the remote node granted an emergency service access to the at least one imaging system of the local imaging node allowing to execute remote servicing omitting approval requests. A critical alert may refer to an alert, but with a high urgency status, i.e. a notification placed on high priority. This alert can be in different forms, such as audio (e.g., audio signal), visual (e.g., flashing screen), audio-visual (e.g., audio signal with flashing screen), haptic (e.g., vibration on the peripheral devices of the operator of the remote node or on a phone), etc. It is to be understood that the "emergency service access" refers to the ability for emergency responders (such as remote operator of a remote node) to quickly and effectively respond to an issue in times of crisis/potential crisis. This is a crucial for ensuring that changes are made proactively in such an event if there is e.g., an accident at the local node. In this case, the operator of the remote node is granted an access without any approvals, so that critical time can be saved in order to make the required changes as soon as possible in order to mitigate the possible effects, prevent bigger challenges with the imaging machine, which will disrupt the workflow in the hospital, leading to bigger technical faults and failures, which will be costly to repair and which will require changing the clinical workflow, both leading to substantial financial losses.

In some embodiments, the least one control interface comprises at least one of the following or the combination of at least one of the following: keyboard, mouse, trackpad, touchscreen display; and wherein establishing the two-way secure communication channel comprises using any one or the combination of: virtual private network (VPN), zero trust network access (ZTNA), secure access service edge (SASE), software-defined perimeter (SDP), software-defined wide-area networks (SD-WANs), identity and access management (IAM), privileged access management (PAM), unified endpoint management (UEM), virtual desktop infrastructure (VDI), secure web gateway (SWG), cloud access security brokers (CASBs). Certain protocols like VPN provide one set of advantages (like secure peer-to-peer communication), while other protocols provide additional advantages, such as multi-site/multi-party customizable protocol. Hence, in certain cases different protocols are preferred to be used, or a combination of different protocols are preferred to be used in the present disclosure in order to combine advantages of multiple protocols and establishing an improved two-way secure communication channel in clinical environment.

In another aspect, a system, method, computer program and non-transitory computer-implemented medium for remote servicing and technical support of imaging equipment is disclosed, wherein the method comprises obtaining information from a local node by an operator of the local node, the local node comprising at least one imaging system, establishing a two-way secure communication channel with an operator of a remote node, wherein the operator of the local node establishes the requirements for such a session and requests an identity of an operator of the remote node, confirming the identity of the operator of the remote node with a database of operators, granting access to the local node if the identity matches, granting access to the local node's imaging system peripheral devices to execute changes to the local node's imaging system, wherein the operator of the local node approves such changes.

Establishing a two-way secure communication channel between a local and remote node operator ensures that sensitive data related to the imaging system is protected during remote servicing. This secure channel prevents unauthorized access and maintains the integrity of the transmitted information, which is crucial in healthcare settings where patient data and system functionality are involved.

By confirming the identity of the remote operator through a database, the method ensures that only authorized personnel can access the local node's imaging system. This step enhances security by preventing unauthorized interventions, thereby safeguarding the imaging equipment and maintaining compliance with privacy regulations.

Granting access to the local node's imaging system peripheral devices allows for remote execution of necessary changes, which can streamline maintenance processes and reduce the need for on-site visits. This capability is particularly beneficial in healthcare environments where timely equipment servicing is essential to avoid disruptions in patient care.

At least in some embodiments, the system incorporates authentication and authorization techniques to ensure secure access. Remote users confirm their identity through a database, and access is granted upon approval. This process may enhance security by preventing unauthorized interventions and safeguarding the system. The system also includes a logging mechanism to record remote interactions for auditing and compliance purposes, ensuring accountability and traceability of actions performed during the session.

At least in some embodiments, the system supports integration with existing hospital information systems, facilitating seamless data exchange and workflow management. This integration allows for remote diagnostics, system health checks, and maintenance activities, reducing the need for on-site visits. The system's design ensures that sensitive data remains protected, maintaining the integrity of transmitted information, which is significant in healthcare settings where patient data and system functionality are involved.

At least in some embodiments, establishing a two-way secure communication channel between a local and remote node operator ensures that sensitive data related to the imaging system is protected during remote servicing. This secure channel prevents unauthorized access and maintains the integrity of the transmitted information, which is crucial in healthcare settings where patient data and system functionality are involved. By confirming the identity of the remote operator through a database, the method ensures that only authorized personnel can access the local node's imaging system. This step enhances security by preventing unauthorized interventions, thereby safeguarding the imaging equipment and maintaining compliance with privacy regulations. Granting access to the local node's imaging system peripheral devices allows for remote execution of necessary changes, which can streamline maintenance processes and reduce the need for on-site visits. This capability is particularly beneficial in healthcare environments where timely equipment servicing is essential to avoid disruptions in patient care.

At least in some embodiments, the system provides for a method and medium for secure remote serviceability of imaging equipment without relying on traditional VPNs or third-party remoting software. By utilizing a communication protocol, such as WebRTC protocol, the system establishes a two-way secure communication channel that facilitates real-time sharing of screens, keyboard, and mouse inputs. This approach addresses security concerns associated with existing methods and offers a streamlined solution for remote maintenance. Additionally, the integration of a virtual camera module for screen capture and the use of authentication and authorization techniques enhance the security and efficiency of remote access. The system's ability to support multiple local systems and provide a comprehensive logging mechanism for auditing purposes further distinguishes it from prior art, offering a robust and compliant solution for healthcare settings. At least in some embodiments, the integration of a virtual camera module for screen capture and the use of authentication and authorization techniques enhance the security and efficiency of remote access. The system's ability to support multiple local systems and provide a comprehensive logging mechanism for auditing purposes further distinguishes it from prior art, offering a robust and compliant solution for healthcare settings.

In one embodiment, the system for remote servicing and technical support of imaging equipment involves a local node equipped with an imaging system that can be accessed remotely through a secure communication channel. This channel is established using WebRTC technology, ensuring encrypted data transmission between the local and remote nodes. The local node operator initiates the session by obtaining system information and setting session requirements, including verifying the identity of the remote operator through a database check. Once verified, the remote operator is granted access to the local node's imaging system and peripheral devices, such as scanners or printers, to perform necessary changes. The local operator retains control by approving any modifications made by the remote operator.In another embodiment, the system includes a virtual camera module that captures the local node's screen, converting the screen into a media stream for transmission. This stream is rendered on a web application at the remote node, allowing the remote operator to interact with the local system using keyboard and mouse inputs. These inputs are emulated on the local system, enabling real-time control. Additionally, the system can be configured to allow multiple remote operators to join the session, provided their identities are verified and approved by the local operator. This flexibility ensures that the system can adapt to various operational needs while maintaining security and control.

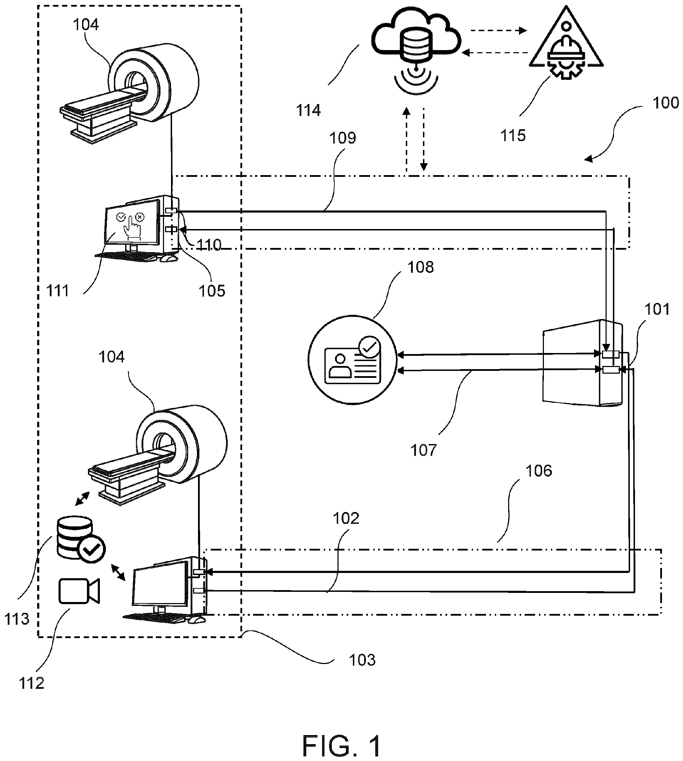

Further, the embodiments will be further discussed with respect to figures. Fig. 1 depicts a first embodiment according to the invention. In this embodiment, a computer-implemented method or a computer-readable medium, or a system for remote servicing and technical support. In one aspect, a computer-implemented method (100) for remote servicing and technical support of imaging system is described, the method comprising: Obtaining (101) operational information (102) of a local node (103), the local node comprising at least one imaging system (104) and at least one control interface (105); Establishing a two-way secure communication channel (106) between an operator of a remote node and an operator of the local node, wherein an identity confirmation request (107) of the operator of the remote node is performed; Confirming (108) the identity of the operator of the remote node through the identity confirmation request (107), transmitting (109) the operational information of the local node to the remote node and granting remote service access to the local node, the remote service access to the local node comprises granting access to at the at least one control interface (110) allowing to execute remote servicing to the at least one imaging system, wherein an approval request (111) of such remote servicing execution is displayed to the local operator.

In some embodiments, any one of or combination of the following devices are used for execution of the identity confirmation request of the operator of the remote node: camera (112), fingerprint sensor, ID scan device, RFID scan device, database verification unit (113), knowledge-based authentication system. In some embodiments establishing the two-way secure communication channel (106) comprises confirmation by an administrator and/or the local operator of a remote service access request, and wherein each confirmed and unconfirmed remote service access request is stored in a service database (114) as a log (115) for further review by the administrator and/or the local operator.

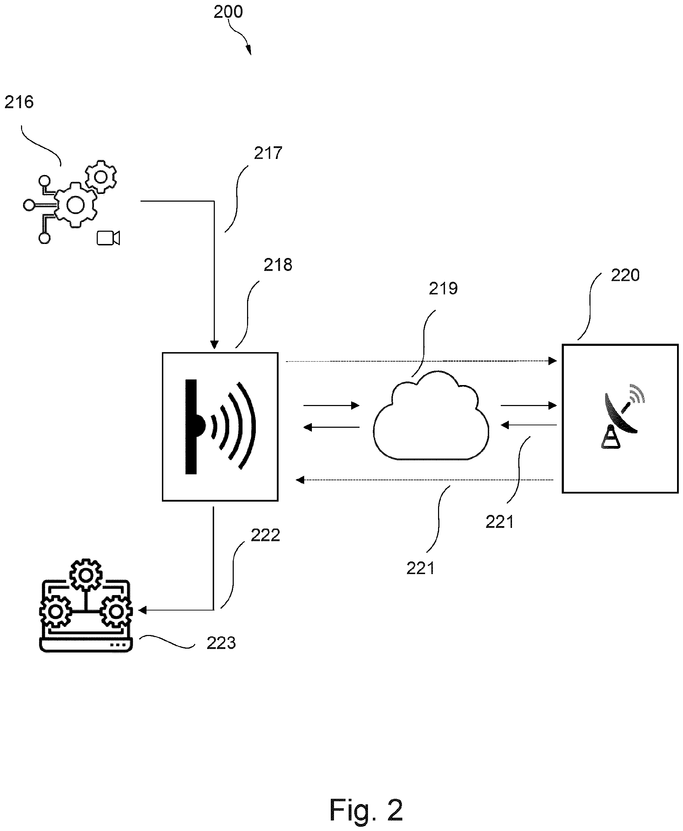

Fig. 2 depicts another embodiment further detailing the aspects of the invention. It is to be understood that reference to any of the components described is purely exemplary and other systems can be used to describe the embodiment. According to this embodiment, a virtual camera module (216) is generating a media event (217) and optionally transmitting it to a transmitter module (218). As a an example, the generation of the media event (217) might comprise rendering of the system screen (e.g., publishing of the local screen) and transmitting to the transmitter module (218). As a practical example, the transmitter module could be a RF (radiofrequency) transmitter module, such as a small PCB (printed circuit board) sub-assembly capable of transmitting a radio wave and modulating that wave to carry data, such as the rendered system screen data or another media event (-s) (217). Transmitter module (218) could be implemented in addition to a microcontroller which will provide data to the module which can be transmitted. Optionally, the media event can be transmitted through a WebRTC channel (219). The added advantage of using the WebRTC channel (219) is that the media event (e.g., published local screen with attached video track) could be subscribed (e.g., increase protection of the data transmitted via the WebRTC channel) before being transmitted to the remote node. In some embodiments, the media events are being transmitted from the WebRTC channel to the receiver module (220) and/or the media events are being transmitted directly from the transmitted module (218) to the receiver module (220). The receiver module of the local node is configured for receiving the media events (217), processing the media events (217) and transmitting the media events, and sending the K/M (keyboard/mouse) events, which enable the screen control and remote servicing. The K/M events can be transmitted (221) to the WebRTC channel, or directly to the transmitter module. The transmitter module is listening to the K/M events, which enables the screen control and/or access to the remote servicing. In some embodiments, the local nodes' transmitter module (or the local node in general) is configured to emulate (222) interface inputs that are being performed on the remote node and/or the receiver module. As an example, such interface inputs can comprise the K/M events generated by the e.g., receiver module (220). In some embodiments, the emulated inputs (222) can be transmitted to an operating system module (223), which can control the local node and/or perform changes to the local node, such as performing remote servicing. As a practical example, the operating system module could be a Win32 module Systeminteraction, which allows to interact with the system. In other embodiments, emulated inputs (222) interact with other modules that allow to make the system changes, such as changes to the imaging device and/or perform the remote servicing. As another practical example, both the receiver and transmitter module are just processors configured for providing the functionality described above, wherein the processors can be general-type processors, or specific processors with specific hardware and software linked and/or embedded to the processors, that allows to perform the functionality described above. Furthermore, use of the WebRTC channel allows for near real-time secure exchange of information.

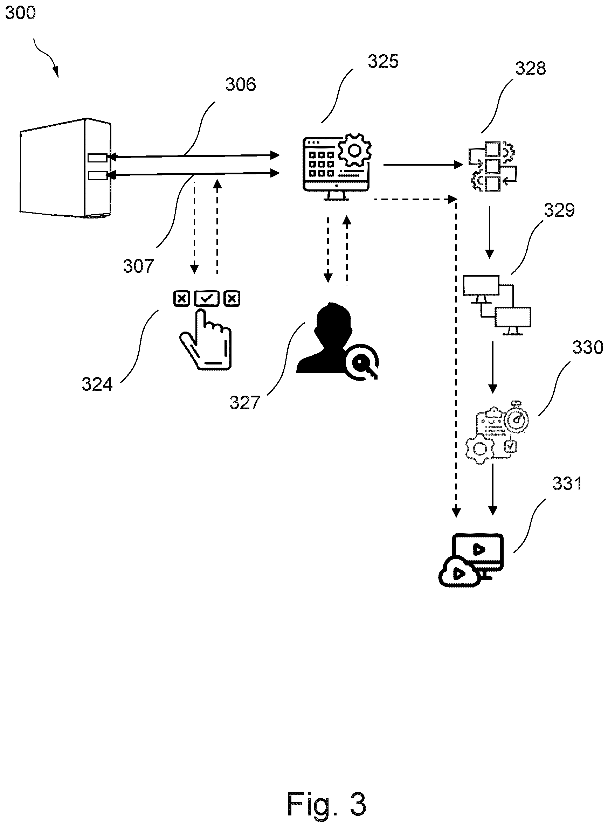

Fig. 3 depicts a further embodiment according to the invention. The mentioned further embodiment describes an example of the approval flow and session workflow that could be implemented within the current invention. For clarity, the steps of obtaining the operational information are not described in this embodiment, but these steps can still be implemented at least in some embodiments. According to the embodiment of Fig. 3, the user of the system logs in to the remote node of the system and sends an identity confirmation request (307) through the secure communication channel (306). At least in some embodiments, the confirmation request (307) comprises selecting rooms in the hospital workflow (324) from the operational information (302), wherein the selecting of rooms in the hospital workflow (324) may comprise additional the information on the equipment of the local node (303) and the workflow information, such as list of rooms, list of imaging sites, list of imaging equipment, scheduling time, workflow information, time-slots allocated for different examinations according to a certain imaging system. At least in some embodiments, the confirmation request (307) additionally comprises selecting and/or invoking the workflow of the hospital at least in relation to some of the rooms in the local nodes' setting. The user additionally may have the possibility of choosing a certain timeslot in the workflow and requesting control specifically on that timeslot. Then the confirmation request (307) may be transmitted to an administration application (325), wherein the operator of the local node is being notified through e.g., the Graphical User Interface and/or the local screen on the request (307). The operator of the local node, such as the hospital administrator, is logging in the system (327) upon receiving such a request and approving such a request. The operator of the local node also has the possibility of reviewing and amending (327) the request, such as amending the privileges given to the remote user upon the request (307), amending the access to certain imaging systems of the local node (303), etc. It is to be understood that the identity confirmation request may comprise multiple requests coming from different users, and one request is provided only exemplary. Upon approval of the request, a session workflow (328) might be started at least in some embodiments. The session workflow comprises of a log-in of the local user to the local node and acquiring access to the local nodes at least one imaging system (304) and at least one control interface (305), wherein optionally the operator of the remote node is requesting for approval to take control of the local node, and upon such approval, connecting to the service level access in the local node. At least in some embodiments, the remote service access comprises sharing the screen of the local system (329) and which is shared with the operator of the remote node, such as the remote service engineer that is performing the remote servicing. At least in some embodiments, the operator of the remote node has access to control interface of the local imaging system and/or access to the screen of the local system in order to control the local nodes' imaging system. At least in some embodiments, the operator of the remote node initiates a streaming and workflow event (330), wherein the event (330) is sent to the streaming container (331). The streaming container (331) is configured to stream workspace directly to web browser on any device and from any location, such mobile devices (e.g., tablet) in the remote node, display of the control system of the remote node and other suitable means. In some embodiments, a launch event is performed upon the initialization of the software. It is to be understood that the present embodiment is merely exemplary and other embodiments could be described describing a possible workflow of the invention according to the present invention.

In some embodiments, the system automatically logs out the remote operator after a predetermined period of inactivity to enhance security.

In some embodiments, the local node operator can terminate the remote session at any time, ensuring control over the access to the imaging system.

In some embodiments, the remote operator's access is logged and monitored for auditing and compliance purposes, ensuring accountability and traceability of actions performed during the session.

In some embodiments, a secure communication channel is established using end-to-end encryption to ensure data confidentiality and integrity during transmission.

In some embodiments, the local node operator can customize access permissions for the remote operator based on specific tasks or system areas.

In some embodiments, the system provides real-time alerts to the local operator for any unauthorized access attempts during the remote session.

In some embodiments, the remote operator can access diagnostic tools within the local node to perform system health checks and generate reports.

In some embodiments, the remote operator's access is restricted to read-only mode unless explicitly granted write permissions by the local operator.

In some embodiments, the local node includes a logging mechanism to record all remote interactions for future review and analysis.

In some embodiments, the remote operator can initiate software updates on the local node's imaging system, subject to approval by the local operator.

In some embodiments, the remote operator can collaborate with multiple local operators simultaneously through a shared communication platform.

In some embodiments, the system supports integration with existing hospital information systems for seamless data exchange and workflow management.

In some embodiments, the remote operator can schedule maintenance tasks to be executed automatically at predefined times.

In some embodiments, the system provides a user interface for configuring access levels and permissions for remote operators. The interface can be located in a local node, remote node or combination of the two.

In some embodiments, the remote operator can view real-time performance metrics of the local node's imaging system during the session.

In some embodiments, the system includes a fail-safe mechanism to revert changes made during the remote session if unauthorized modifications are detected.

In some embodiments, the remote operator can generate and export detailed reports of the maintenance activities performed during the session.

In some embodiments, the system allows for remote training sessions by enabling screen sharing and interactive guidance tools.

In some embodiments, the remote operator can customize the user interface layout to prioritize specific system metrics and controls.

In some embodiments, the system supports role-based access control to tailor permissions based on the operator's role and responsibilities.

In some embodiments, the remote operator can access historical data logs to analyze past system performance and maintenance activities.

In some embodiments, the system provides notifications to the local operator for any updates or changes made during the remote session.

In some embodiments, the remote operator can utilize a virtual whiteboard for collaborative troubleshooting with the local operator.

In some embodiments, the system includes a feature to log and timestamp all remote access sessions for compliance and auditing purposes.

In some embodiments, the remote operator can receive real-time feedback on system diagnostics and performance during the session.

In some embodiments, the operator of the local system can automatically adjust access permissions based on the remote operator's location and network security level.

In some embodiments, the remote operator can initiate a secure video call with the local operator for enhanced communication during the session.

In some embodiments, the system supports integration with third-party diagnostic tools to enhance remote troubleshooting capabilities.

In some embodiments, the remote operator can configure alerts for specific system events to receive timely notifications.

In some embodiments, the remote system includes a dashboard for monitoring multiple imaging systems simultaneously, providing a comprehensive overview of their status and performance.

In some embodiments, the remote operator can access a secure log of all interactions and changes made during the session for review and compliance purposes.

In some embodiments, the system can dynamically allocate bandwidth to optimize the performance of the remote session based on network conditions.

In some embodiments, the remote operator can remotely calibrate the imaging system to ensure optimal performance and accuracy.

In some embodiments, the system provides end-to-end encryption to protect data integrity and confidentiality during remote sessions.

In some embodiments, the remote operator can schedule automated diagnostics to run at specified intervals for proactive system maintenance.

In some embodiments, the remote operator can initiate a secure file transfer to update software or upload necessary documents to the local node.

In some embodiments, the system includes automated alerts for potential security breaches during remote sessions.

In some embodiments, the remote operator can utilize augmented reality tools to assist in troubleshooting and maintenance tasks.

In some embodiments, the system allows for remote configuration of imaging system settings to optimize performance based on specific use cases.

In some embodiments, the system includes a real-time analytics engine to provide insights into system usage and performance trends.

In some embodiments, the system can automatically generate alerts for scheduled maintenance based on usage patterns and system diagnostics.

In some embodiments, the system integrates with cloud-based storage solutions for secure data backup and retrieval.

In some embodiments, the system supports integration with artificial intelligence tools to enhance predictive maintenance capabilities.

In some embodiments, wherein the system includes means for real-time collaboration between multiple remote operators for complex troubleshooting tasks.

In some embodiments, the system allows for remote configuration of imaging system settings to optimize performance based on specific use cases.

In some embodiments, the system includes a real-time analytics engine to provide insights into system usage and performance trends.

In some embodiments, the system includes means for remote diagnostics to identify and resolve issues before they impact system performance.

In some embodiments, the system provides a secure channel for exchanging encrypted messages between local and remote operators.

In some embodiments, the system can automatically generate alerts for scheduled maintenance based on usage patterns and system diagnostics.

The above-described is illustrory in nature and non-limiting in any way. Combinations of the previous embodiments are envisioned within the scope of the present application even if not explicitly described. As was previously described, the embodiments described previously at least in some instances describe a system for enabling remote serviceability over the internet. At least in some instances, this may provide for a system with secure communication and screen sharing without relying on traditional VPNs or third-party remoting software. The system may employ WebRTC at least in some embodiments for communication, a virtual camera for screen capture, and modules for keyboard and mouse emulation. Following are some non-limiting practical examples of how the system according to the present application can be used:

As an example, the system and method described herein can be used as a secure remote serviceability system.A system enabling remote serviceability of hardware systems over the internet using a secure communication channel established via communication protocols such as WebRTC is provided at least in some embodiments. The system may comprise a transmitter module that captures and transmits screen data using a virtual camera, and a receiver module that receives and renders the screen data on a remote user's application layout. As an advantage, this embodiment would address the need for secure remote access without using traditional VPNs or third-party software.

As an example, the system and method described herein can be used as a virtual camera screen capture method and system. A system for capturing a local system's screen using a virtual camera module, converting the captured screen into a media stream, and transmitting the stream over a WebRTC communication channel is provided at least in some embodiments. This technique allows for secure and efficient screen sharing in remote serviceability applications.

As an example, the system and method described herein can be used asa system for keyboard and mouse emulation for remote control. A system for emulating keyboard and mouse inputs on a local system based on inputs received from a remote user via a WebRTC communication channel is provided at least in some embodiments. The method includes encrypting the inputs and using native modules to emulate the inputs on the local system, enabling remote control of the system.

As an example, the system and method described herein can be used as a system for authentication and authorization workflow management.A system for managing remote serviceability access through an authorization workflow is provided at least in some embodiments. The system includes a process for submitting and approving remote access requests, ensuring that only authorized users can initiate remote service sessions. This workflow enhances security and compliance in remote serviceability applications.

As an example, the system and method described herein can be used as a system formulti-system remote access selection. A system that allows a remote user to select and access multiple local systems or screens through a secure communication channel is provided at least in some embodiments. The method includes a user interface for selecting specific systems and managing access permissions, providing flexibility in remote serviceability scenarios.

WebRTC, or Web Real-Time Communication, is a technology that enables real-time communication over the internet. WebRTC allows audio, video, and data sharing directly between web browsers without requiring additional plugins or software. WebRTC facilitates peer-to-peer connections, making WebRTC possible to conduct video calls, voice chats, and data transfers seamlessly. WebRTC operates by establishing a direct connection between two devices, enabling them to exchange media and data streams. This process involves several components: media capture, peer connection, data channels, signalling, security.

A "node" refers to a point in a network where data can be created, received, or transmitted. It typically represents a device or system, such as a computer or server, that participates in a network communication process. In present case, it involves local and remote nodes interacting for remote servicing and technical support, particularly in healthcare technology settings.

A two-way secure communication channel is a communication link that allows data to be exchanged in both directions between two parties while ensuring the confidentiality, integrity, and authenticity of the transmitted information. This is typically achieved through encryption and authentication protocols, such as those provided by WebRTC, to prevent unauthorized access and ensure that only authorized users can participate in the communication.

An imaging system refers to a combination of hardware and software components designed to capture, process, and display images. In the context of healthcare, it often involves devices such as X-ray machines, MRI scanners, or ultrasound equipment, which are used to create visual representations of the interior of a body for clinical analysis and medical intervention. These systems are crucial for accurate diagnosis and treatment planning.

Near real-time data exchange" refers to the rapid transmission and processing of data with minimal delay, allowing information to be shared almost instantaneously between systems or users. This capability is essential for applications requiring timely updates and interactions, such as remote monitoring and control in healthcare settings.

Granting access to a local node's imaging system involves authorizing a remote operator to interact with and make necessary changes to the imaging equipment. This process ensures that the remote operator can perform maintenance or support tasks while maintaining security protocols. Access is typically controlled through authentication and approval by the local operator, ensuring that only authorized personnel can modify the system.

The processes and displays presented herein are not inherently related to any particular computer or other apparatus. Various general-purpose systems may also be used with programs in accordance with the teachings herein, or it may prove convenient to construct more specialized apparatus to perform one or more method steps. The structure for a variety of these systems is discussed in the description below. In addition, any programming language that is sufficient for achieving the techniques and implementations of the present disclosure may be used. In addition, the language used in the specification has been principally selected for readability and instructional purposes and may not have been selected to delineate or circumscribe the disclosed subject matter. Accordingly, the present disclosure is intended to be illustrative, and not limiting, of the scope of the concepts discussed herein.

Aspects of the disclosure may be supported by various information technology (IT) backends, including either or both local architectures, either as monoliths, networked, or a combination thereof, and hosted architectures, such as a software as a service (SaaS), platform as a service (PaaS), and/or infrastructure as a service (IaaS), or the like. In an example, a supporting infrastructure includes multiple interconnected layers respectively hosting, as an abstraction, various IT process, service, account, and other management components. The layers of the supporting infrastructure may be divided into, for example and without imputing limitation, a Foundation Layer (FL), a Clinical Storage Layer (CSL), a Platform Layer (PL), and a Cloud Provision Layer (CPL). Each layer is generally structured to support a designated aspect of the IT backend supporting software product offerings and is made of various components that each may include any or all of libraries, functions, application programming interfaces (APIs), data stores, and more. The FL includes hosting management utilities enabling management of accounts related to the CPL, either directly by an end user or by a software product IT management. The DL serves as an environment for testing and development software products provided over the IT backend. The CPL is a foundational layer "upon" which the FL, DL, and PL operate and with which components of each of the other layers interact to varying degrees. The CPL provides direct access to remote server infrastructure supporting the IT backend supports the infrastructural hosting (e.g., spinning up of virtual machines, containers, etc.) for supported applications. The PL includes a number of components for providing platform support for software products. Platform support may include functions such as orchestration, layer integrations, software product operational functions, data repository access and/or management, and more. The CPL includes various processes for performing infrastructural services supporting the layers which operate upon it. In some examples, these processes may include virtual server instantiation, hardware (e.g., physical server) orchestration and management, redundancy and backup operations, security and logging, routing operations, and more. The PL is the primary location of the hosted product and service offerings, as well as the processes that support these offerings, including, but not limited to, providing services to users. Among the various processes and modules that reside in, or originate from, the PL, orchestration and management modules typically interconnect the PL and the CPL.

In some embodiments, the confirmation of the remote operator may be performed through an Identity and Authentication Management (IAM) module that may provide the sign on and authentication processes enabling the above-described features. IAM exposes representational state transfer (REST) application programming interfaces (APIs) and/or hypertext transfer protocol secure (HTTPS) access points which applications, such as <>, which are installed at least in part at a local, or "on premise" or "on prem," computer may utilize for sign on access, such as through a login page or the like. Various on prem as well as mobile devices are able to utilize IAM through REST API access, including, for example and without imputing undue limitation, a medical diagnostic device, a mobile device, and a laptop computer.

REST APIs and/or HTTPS access points are provided as REST endpoints. Here, medical diagnostic device, mobile device, and laptop computer access REST endpoints across a communications network (e.g., LAN, WAN, the internet, mobile network, etc.). REST endpoints allow for access to a variety of processes, and may include an API signing and identity management processes for storing public and private key pairings, among other features. In some examples, other processes provided by IAM may include, without limitation, two-factor authentication, role-based access control, single sign on (SSO), device propositions, multi-tenancy, and more. Moreover, authentication, authorization, and identity management, as well as policy decision point processes, may manage login authentication and policy management (e.g., evaluation, inspection, etc.). Further, identity management may be responsible for synchronize policies and maintaining synchronization of such for user accounts.

REST APIs and/or HTTPS access points may allow for applications to provide to users interfaces for configuration and user preferences and/or receiving or viewing alerts and/or notices from various processes in PL. A logging and auditing module provides for operational records of events and processes within FL, which may be later retrieved and reviewed. Notably, in some examples, IAM may integrate across FL and CPL, such as, for example and without imputing undue limitation, when a product or service includes direct instantiation of a cloud managed service or other CPL feature. PL is the primary layer of the supporting infrastructure in which hosted business logic and platform services are conducted. PL includes a managed platform services module, an orchestration module, and a Platform as a Service (PaaS) manager module, though it is to be understood that PL may include additional modules and/or processes relevant to product and service offerings operating upon the backend infrastructure. PaaS manager likewise intersects with CPL via cloud managed services. PaaS manager 228 handles platform-level workload orchestration, such as in the case of a hosted singular platform that serves customers rather than separate applications respectively serving individual customers. In addition, PaaS manager 228 performs orchestration operations for managed platform services 210.

Terms are to be associated with the appropriate physical quantities and are merely convenient labels applied to these quantities. Unless specifically stated otherwise as apparent from the following discussion, it is appreciated that throughout the description, discussions utilizing terms such as "processing" or "computing" or "calculating" or "determining" or "displaying" or the like, refer to the action and processes of a computer system, or similar electronic computing device, that manipulates and transforms data represented as physical (electronic) quantities within the computer system memories or registers or other such information storage devices. Portions of the present disclosure include processes and instructions that may be embodied in software, firmware, or hardware, and when embodied in software, may be downloaded to reside on and be operated from different platforms used by a variety of operating systems.

The illustrations of the embodiments described herein are intended to provide a general understanding of the structure of the various embodiments. The illustrations are not intended to fully describe all the elements and features of the disclosure described herein. Many other embodiments may be apparent to those of skill in the art upon reviewing the disclosure. Other embodiments may be utilized and derived from the disclosure, such that structural and logical substitutions and changes may be made without departing from the scope of the disclosure. Additionally, the illustrations are merely representational and may not be drawn to scale. Certain proportions within the illustrations may be exaggerated, while other proportions may be minimized. Accordingly, the disclosure and the figures are to be regarded as illustrative rather than restrictive.

In accordance with various embodiments of the present disclosure, the methods described herein may be implemented using a hardware computer system that executes software programs. Further, in exemplary implementations can include distributed processing, component/object distributed processing, and parallel processing. Virtual computer system processing may implement one or more of the methods or functionalities as described herein, and a processor described herein may be used to support a virtual processing environment.

The illustrations of the examples described herein are intended to provide a general understanding of the structure of the various embodiments. The illustrations are not intended to fully describe all the elements and features of the disclosure described herein. Many other examples may be apparent to those of skill in the art upon reviewing the disclosure. Other embodiments may be utilized and derived from the disclosure, such that structural and logical substitutions and changes may be made without departing from the scope of the disclosure. Additionally, the illustrations are merely representational and may not be drawn to scale. Certain proportions within the illustrations may be exaggerated, while other proportions may be minimized. Accordingly, the disclosure and the figures are to be regarded as illustrative rather than restrictive. Any subsequent arrangement designed to achieve the same or similar purpose may be substituted for the specific examples shown. This disclosure is intended to cover all subsequent adaptations or variations of various embodiments. The appended claims are intended to cover all such modifications, enhancements, and other embodiments which fall within the scope of the present disclosure. Thus, to the maximum extent allowed by law, the scope of the present disclosure is to be determined by the broadest permissible interpretation of the following claims and their equivalents and shall not be restricted or limited by the foregoing detailed description.

Some portions of the description are presented in terms of symbolic representations of operations on non-transient signals stored within a computer memory. These descriptions and representations are used by those skilled in the data processing arts to convey the substance of their work most effectively to others skilled in the art. Such operations typically require physical manipulations of physical quantities. Usually, though not necessarily, these quantities take the form of electrical, magnetic, or optical signals capable of being stored, transferred, combined, compared, and otherwise manipulated. It is convenient at times, principally for reasons of common usage, to refer to these signals as bits, values, elements, symbols, characters, terms, numbers, or the like. Furthermore, it is also convenient at times to refer to certain arrangements of steps requiring physical manipulation of physical quantities as modules or code devices, without loss of generality.

However, all of these and similar terms are to be associated with the appropriate physical quantities and are merely convenient labels applied to these quantities. Unless specifically stated otherwise as apparent from the following discussion, it is appreciated that throughout the description, discussions utilizing terms such as "processing" or "computing" or "calculating" or "determining" or "displaying" or the like, refer to the action and processes of a computer system, or similar electronic computing device, that manipulates and transforms data represented as physical (electronic) quantities within the computer system memories or registers or other such information storage devices. Portions of the present disclosure include processes and instructions that may be embodied in software, firmware, or hardware, and when embodied in software, may be downloaded to reside on and be operated from different platforms used by a variety of operating systems.

In a networked deployment, the computer system operates in the capacity of a server, or as a client user computer in a server-client user network environment, or as a peer computer system in a peer-to-peer or distributed network environment. The computer system can also be implemented as or incorporated into various devices, such as a server or another type of computer such as a workstation that includes a controller, a stationary computer, a mobile computer, a personal computer (PC), a laptop computer, a tablet computer, or any other machine capable of executing a set of software instructions sequentially or non-sequentially that specify actions to be taken by that machine. The computer system can be incorporated as an integrated system part of a larger system that includes additional devices. In an embodiment, the computer system can be implemented using electronic devices that provide voice, video, or data communication possibilities. Further, while the computer system is illustrated in the singular, the term "system" shall also be taken to include any collection of systems or sub-systems that individually or jointly execute a set or multiple sets, of software instructions to perform one or more computer functions.

The computer system may also include a processor. The processor executes instructions to implement some, or all aspects of methods and processes described herein. The processor is tangible and non-transitory. As used herein, the term "non-transitory" is to be interpreted not as an eternal characteristic of a state, but as a characteristic of a state that will last for a period. The term "non-transitory" specifically disavows fleeting characteristics such as characteristics of a carrier wave or signal or other forms that exist only transitorily in any place at any time. The processor is an article of manufacture and/or a machine component. The processor is configured to execute software instructions to perform functions as described in the various embodiments herein. The processor may be a general-purpose processor or may be part of an application specific integrated circuit (ASIC). The processor may also be a microprocessor, a microcomputer, a processor chip, a controller, a microcontroller, a digital signal processor (DSP), a state machine, or a programmable logic device, a logical circuit, including a programmable gate array (PGA), such as a field programmable gate array (FPGA), or another type of circuit that includes discrete gate and/or transistor logic. The processor may be a central processing unit (CPU), a graphics processing unit (GPU), or both. Additionally, any processor described herein may include multiple processors, parallel processors, or both. Multiple processors may be included in, or coupled to, a single device or multiple devices. The processor can include one or more internal levels of cache, and a bus controller or bus interface unit to direct interaction with a bus. The term "processor" as used herein encompasses an electronic component able to execute a program or machine executable instruction. References to a computing device comprising "a processor" should be interpreted to include more than one processor or processing core, as in a multi-core processor. A processor may also refer to a collection of processors within a single computer system or distributed among multiple computer systems. The term computing device should also be interpreted to include a collection, or network, of computing devices each including a processor or processors. Programs have software instructions performed by one or multiple processors that may be within the same computing device or which may be distributed across multiple computing devices. Further, the software instructions, when executed by the processor, perform one or more steps of the methods and processes as described herein.

The computer system further includes a main memory and a static memory, where memories in the computer system communicate with each other and the processor via a bus. Either or both main memory and the static memory may be considered representative examples of the memory of the controller, and store instructions used to implement some, or all aspects of methods and processes described herein. Memories described herein are tangible storage mediums for storing data and executable software instructions and are non-transitory during the time software instructions are stored therein. The main memory and the static memory are articles of manufacture and/or machine components. The main memory and the static memory are computer-readable mediums from which data and executable software instructions can be read by a computer (or e.g., the processor). Each of the main memory and the static memory may be implemented as one or more of random access memory (RAM), read only memory (ROM), flash memory, electrically programmable read only memory (EPROM), electrically erasable programmable read-only memory (EEPROM), registers, a hard disk, a removable disk, tape, compact disk read only memory (CD-ROM (Compact Disk - Read Only Memory)), digital versatile disk (DVD), floppy disk, Blu-ray disk, or any other form of storage medium known in the art. The memories may be volatile or non-volatile, secure and/or encrypted, unsecure and/or unencrypted.

"Memory" is an example of a computer-readable storage medium. Computer memory is any memory which is directly accessible to a processor. Examples of computer memory include RAM memory, registers, and register files. References to "computer memory" or "memory" should be interpreted as possibly being multiple memories. The memory may for instance be multiple memories within the same computer system. The memory may also be multiple memories distributed amongst multiple computer systems or computing devices. The memory may store various software applications including computer executable instructions, that when run on the processor, implement the methods and systems set out herein. Other forms of memory, such as a storage device and a mass storage device, may also be included and accessible by the processor (or processors) via the bus. The storage device and mass storage device can each contain any or all of the methods and systems discussed herein.

The computer system can further include a communications interface by way of which the computer system can connect to networks and receive data useful in executing the methods and system set out herein as well as transmitting information to other devices. The computer system further includes a video display unit as an output device by which information can be output, such as a liquid crystal display (LCD), an organic light emitting diode (OLED), a flat panel display, a solid-state display, or a cathode ray tube (CRT), for example. Additionally, the computer system includes an input device, such as a keyboard/virtual keyboard or touch-sensitive input screen or speech input with speech recognition, and a cursor control device, such as a mouse or touch-sensitive input screen or pad. The computer system also optionally includes a disk drive unit, a signal generation device, such as a speaker or remote control, and/or a network interface device.

The processes and displays presented herein are not inherently related to any particular computer or other apparatus. Various general-purpose systems may also be used with programs in accordance with the teachings herein, or it may prove convenient to construct more specialized apparatus to perform one or more method steps. The structure for a variety of these systems is discussed in the description below. In addition, any programming language that is sufficient for achieving the techniques and implementations of the present disclosure may be used. In addition, the language used in the specification has been principally selected for readability and instructional purposes and may not have been selected to delineate or circumscribe the disclosed subject matter. Accordingly, the present disclosure is intended to be illustrative, and not limiting, of the scope of the concepts discussed herein.

In accordance with various embodiments of the present disclosure, the methods described herein may be implemented using a hardware computer system that executes software programs. Further, in an exemplary, non-limited embodiment, implementations can include distributed processing, component/object distributed processing, and parallel processing. Virtual computer system processing may implement one or more of the methods or functionalities as described herein, and a processor described herein may be used to support a virtual processing environment.

The illustrations of the embodiments described herein are intended to provide a general understanding of the structure of the various embodiments. The illustrations are not intended to fully describe all the elements and features of the disclosure described herein. Many other embodiments may be apparent to those of skill in the art upon reviewing the disclosure. Other embodiments may be utilized and derived from the disclosure, such that structural and logical substitutions and changes may be made without departing from the scope of the disclosure. Additionally, the illustrations are merely representational and may not be drawn to scale. Certain proportions within the illustrations may be exaggerated, while other proportions may be minimized. Accordingly, the disclosure and the figures are to be regarded as illustrative rather than restrictive.

Although specific embodiments have been illustrated and described herein, it should be appreciated that any subsequent arrangement designed to achieve the same or similar purpose may be substituted for the specific embodiments shown. This disclosure is intended to cover all subsequent adaptations or variations of various embodiments. Combinations of the above embodiments, and other embodiments not specifically described herein, will be apparent to those of skill in the art.

The preceding description of the disclosed embodiments is provided to enable any person skilled in the art to practice the concepts described in the present disclosure. As such, the above disclosed subject matter is to be considered illustrative, and not restrictive, and the appended claims are intended to cover all such modifications, enhancements, and other embodiments which fall within the scope of the present disclosure. Thus, to the maximum extent allowed by law, the scope of the present disclosure is to be determined by the broadest permissible interpretation of the following claims and their equivalents and shall not be restricted or limited by the foregoing detailed description.

Other variations to the disclosed embodiments can be understood and effected by those skilled in the art in practicing the claimed invention, from a study of the drawings, the disclosure, and the appended claims. In the claims, the word "comprising" does not exclude other elements or steps. In the claims, the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

### REFERENCE SIGNS

- 101:: obtaining operational information
- 102:: operational information
- 103:: local node
- 104:: at least one imaging system
- 105:: at least one control interface
- 106:: (establishing) a two-way secure communication channel
- 107:: identity confirmation request
- 108:: confirming the identity of the operator of the remote node
- 109:: transmitting the operational information of the local node to the remote node/granting remote service access to the local node
- 110:: granting access to at the at least one control interface allowing to execute remote servicing to the at least one imaging system,
- 111:: an approval request of remote servicing execution is displayed to the local operator.
- 112:: camera
- 113:: database verification unit
- 114:: service database
- 115:: log for further review
- 216:: virtual camera module
- 217:: media event
- 218:: (transmitting to) transmitter module
- 219:: WebRTC channel
- 220:: receiver module
- 221:: K/M events to be transmitted
- 222:: emulate (interface) inputs
- 223:: (transmitted to) an operating system module
- 324:: selecting rooms in the hospital workflow
- 325:: administration application
- 326:: logging in the system
- 327:: reviewing and amending the identity confirmation request
- 328:: session workflow
- 329:: sharing the screen
- 329:: streaming and workflow event
- 330:: streaming container
- 331:: launching event

## Claims

1. A computer-implemented method for remote servicing and technical support of imaging systems, the method comprising:
- Obtaining operational information of a local node, the local node comprising at least one imaging system and at least one control interface;
- Establishing a two-way secure communication channel between an operator of a remote node and an operator of the local node, wherein an identity confirmation request of the operator of the remote node is performed;
- Confirming the identity of the operator of the remote node through the identity confirmation request, transmitting the operational information of the local node to the remote node and granting remote service access to the local node, the remote service access to the local node comprises granting access to at the at least one control interface allowing to execute remote servicing to the at least one imaging system, wherein an approval request of such remote servicing execution is displayed to the local operator.

2. The method of claim 1, wherein the identity confirmation request comprises a comparison with a database of operators, and wherein any one of or combination of the following devices are used for execution of the identity confirmation request of the operator of the remote node: camera, fingerprint sensor, ID scan device, RFID scan device, database verification unit, knowledge-based authentication system.

3. The method of any one of the previous claims, wherein establishing the two-way secure communication channel comprises confirmation by an administrator and/or the local operator of a remote service access request, and wherein each confirmed and unconfirmed remote service access request is stored in a service database as a log for further review by the administrator and/or the local operator.

4. The method of any one of the previous claims, wherein at least one media event is generated and transmitted to the remote node, the media event comprising at least one of: capturing of a screen of the local nodes' imaging device by a virtual camera module, capturing of the screen of the local nodes' imaging device by a camera module, screen scraping of the local screen of the local nodes' imaging device, capturing control interface inputs and emulating such interface inputs to the remote node via an emulation module.

5. The method of any one of the previous claims, wherein the operational information of the local node is transmitted to the operator of the remote node using a WebRTC channel, wherein a near real-time data exchange between the operators of the local and remote nodes is established using the WebRTC channel.

6. The method of any one of the previous claims, wherein the method further includes tracking screen status of the local operator the screen status shared with the operator of the remote node, and wherein the service access to at least one imaging system is executed via least one control interface by the operator of the remote node.

7. The method of any one of the previous claims, wherein the remote service access to the operator of the remote node includes granting remote service access to multiple imaging systems of the local node or multiple affiliated local nodes, wherein each of the multiple imaging systems comprises at least one control interface.

8. The method of any one of the previous claims, wherein further a serviceability unit is initialized, the serviceability unit configured to change the operational settings of the at least one imaging device of the local node and/or of the local node through the two-way secure communication channel.

9. The method of claim 8, wherein the change of the operational settings needs to be approved by operators other than the local operator and the remote operator.

10. The method of any one of the previous claims, wherein the operational information of the local node comprises any one of: service logs, scan information, patient images, service images, reconstruction algorithm information, operator access logs, wherein optionally an analytics module is used for analysing the operational information for potential technical faults of the at least one imaging system of the local node and/or the local node.

11. The method of claim 10, wherein the potential technical faults are visualized or otherwise communicated to at least one of: local operator, remote operator and/or administrator, wherein an instruction for correction of such potential technical faults is provided, wherein upon confirmation of such correction, the system analyses if the potential technical fault is still present, and in the affirmative event, proposes additional corrective actions.

12. The method of any one of the previous claims, wherein the operational information is being continuously monitored, and wherein the remote and/or local operator receive an alert of any possible technical faults that may affect the operation of the at least one imaging system of the local node.

13. The method of any one of claims 12, wherein if critical technical faults are detected, a critical alert is generated to the operator of the remote node, the operator of the remote node granted an emergency service access to the at least one imaging system of the local imaging node allowing to execute remote servicing omitting approval requests.

14. The method of any one of the previous claims, wherein the least one control interface comprises at least one of the following: keyboard, mouse, trackpad, touchscreen display; and wherein establishing the two-way secure communication channel comprises using any one or the combination of: virtual private network (VPN), zero trust network access (ZTNA), secure access service edge (SASE), software-defined perimeter (SDP), software-defined wide-area networks (SD-WANs), identity and access management (IAM), privileged access management (PAM), unified endpoint management (UEM), virtual desktop infrastructure (VDI), secure web gateway (SWG), cloud access security brokers (CASBs).

15. A non-transient computer readable medium containing program instructions for causing a computer to perform the method of:
- Obtaining operational information of a local node, the local node comprising at least one imaging system and at least one control interface;
- Establishing a two-way secure communication channel between an operator of a remote node and an operator of the local node, wherein an identity confirmation request of the operator of the remote node is performed;
- Confirming the identity of the operator of the remote node through the identity confirmation request, transmitting the operational information of the local node to the remote node and granting remote service access to the local node, the remote service access to the local node comprises granting access to at the at least one control interface allowing to execute remote servicing to the at least one imaging system, wherein an approval request of such remote servicing execution is displayed to the local operator.

16. A system with input, output and a processor adapted to obtain operational information of a local node, the local node comprising at least one imaging system and at least one control interface, the processor further configured to establish a two-way secure communication channel between an operator of a remote node and an operator of the local node, wherein an identity confirmation request of the operator of the remote node is performed, and confirm the identity of the operator of the remote node through the identity confirmation request, wherein the processor transmits the operational information of the local node to the remote node and grants remote service access to the local node, the remote service access to the local node comprises granting access to at the at least one control interface and allowing to execute remote servicing to the at least one imaging system.
